# EUROPEAN PATENT APPLICATION

(11) **EP 4 759 335 A1**
(43) Date of publication of application: **17.06.2026**
(21) Application number: 24852234.4
(22) Date of filing: 02.08.2024
(51) Int. Cl.: A61L 9/01, B01D 53/86, B01D 53/00, A61L 101/02

(54) **METHOD FOR PREPARING DEODORANT COMPOSITION AND DEODORANT COMPOSITION PREPARED THEREBY**

(30) Priority: 08.08.2023 KR 20230103418
(71) Applicant: Korea Atomic Energy Research Institute, 34057 Daejeon (KR); GI Co., Ltd., Jeongeup-si, Jeonbuk-do 56212 (KR)
(72) Inventor: CHUNG, Byungyeoup, Daejon 34057 (KR); LEE, Seungsik, Daejon 34057 (KR); KIM, Jinhong, Daejon 34057 (KR); LEE, Sungbeom, Daejon 34057 (KR); BAI, Hyoungwoo, Daejon 34057 (KR); JUNG, Kwangwoo, Daejon 34057 (KR); CHUNG, Moonsoo, Daejon 34057 (KR); JEONG, Gyeonghan, Daejon 34057 (KR); LEE, Hanui, Daejon 34057 (KR); KIM, Tak Hyun, Daejon 34057 (KR); NAM, Halim, Pyeongtaek-si, Gyeonggi-do 17706 (KR); KIM, Sunghyun, Seoul 04793 (KR)
(74) Representative: Cabinet Beau de Loménie
(86) International application number: PCT/KR2024/011442
(87) International publication number: WO 2025/033880

(57) **Abstract**

The present invention relates to a method for preparing a deodorant composition and a deodorant composition prepared thereby, the method comprising a step of irradiating a composite with radiation, the composite comprising a hydroxide of an alkali metal, an oxide of a Group 2 element, an oxide of a Group 4 element, and an oxide of a Group 13 element.

## Description

### [Technical Field]

### CROSS-REFERENCE TO RELATED APPLICATIONS

This application claims the benefit of priority based on Korean Patent Application No. 10-2023-0103418 filed on August 08, 2023, and all contents disclosed in the relevant Korean Patent Application are included as part of the present specification.

### Technical field

The present disclosure relates to a method for preparing a deodorant composition and the deodorant composition prepared thereby.

### [Background Art]

A malodor-causing substance (e.g., formaldehyde, toluene, xylene, ammonia, and benzene) are caused in the daily life or a related industrial field (e.g., a livestock farm, a pig farm, or a poultry farm). The exposure to such a substance may cause various side effects, such as a mild headache, convulsions, or death due to a pulmonary edema, depending on the degree of the exposure.

Meanwhile, to remove the above malodor-causing substance, various types of deodorants conventionally have been suggested. For example, an inorganic oxide complex, which includes a hydroxide of an alkali metal, an oxide of a Group 2 element, an oxide of a Group 4 element, and an oxide of a Group 13 element, has been suggested to remove various malodor-causing substances including formaldehyde, toluene, xylene, ammonia, and benzene.

However, the above-described conventional inorganic oxide complex exhibits an insignificant removal rate, especially, for malodor-causing substances including a sulfur-based compound (e.g., hydrogen sulfide, methyl mercaptan, dimethyl sulfide, or dimethyl disulfide). In addition, the long-term use of the conventional inorganic oxide complex is particularly difficult (e.g., a continuous use for at least one year is difficult).

Accordingly, a deodorant composition, which exhibits an excellent removal rate for a sulfur-based compound, excellent sustainability, and an excellent economic efficiency, is increasingly required.

### [Disclosure]

### [Technical Problem]

An aspect of the present disclosure provides a method for preparing a deodorant composition, which is capable of exhibiting an excellent removal rate for a malodor-causing substance (especially, a malodor-causing substance including a sulfur-based compound), and the deodorant composition prepared thereby.

### [Technical Solution]

According to an aspect of the present disclosure, there are provided a method for preparing a deodorant composition, which includes irradiating a radiation onto a complex including a hydroxide of an alkali metal, an oxide of a Group 2 element, an oxide of a Group 4 element, and an oxide of a Group 13 element, and a deodorant composition prepared thereby.

### [Advantageous Effects]

According to the method for preparing the deodorant composition of the present disclosure, the deodorant composition, which may effectively remove the malodor-causing substance, especially, the sulfur-based compound, in the air.

### [Description of Drawings]

FIGS. 1A and 1B are graphs illustrating removal rates for a hydrogen sulfide by the deodorant composition of Example 1-6, the deodorant composition of Example 2-6, and the complex of Comparative Example 1, over time;
FIGS. 2A and 2B are graphs illustrating removal rates for methyl mercaptan by the deodorant composition of Example 1-6, the deodorant composition of Example 2-6, and the complex of Comparative Example 1, over time;
FIGS. 3A and 3B are graphs illustrating removal rates for a dimethyl sulfide by the deodorant composition of Example 1-6, the deodorant composition of Example 2-6, and the complex of Comparative Example 1, over time;
FIGS. 4A and 4B are graphs illustrating removal rates for a dimethyl sulfide by the deodorant composition of Example 1-6, the deodorant composition of Example 2-6, and the complex of Comparative Example 1, over time;
FIG. 5 is a graph illustrating the removal rate for a hydrogen sulfide depending on the irradiation dose of a gamma ray;
FIG. 6 is a graph illustrating the removal rate for methyl mercaptan depending on the irradiation dose of a gamma ray;
FIG. 7 is a graph illustrating the removal rate for a dimethyl sulfide depending on the irradiation dose of a gamma ray;
FIG. 8 is a graph illustrating the removal rate for a dimethyl sulfide depending on the irradiation dose of a gamma ray;
FIG. 9 is a graph illustrated the content of radicals in a deodorant composition, which is measured through a radical measurement manner using pyrogallol as a substrate, depending on an irradiation dose of an electron beam; and
FIG. 10 is a graph illustrated the content of radicals in a deodorant composition, which is measured through a radical measurement manner using pyrogallol as a substrate, depending on an irradiation dose of a gamma ray.

### [Mode for Invention]

The present disclosure relates to a method for preparing a deodorant composition and the deodorant prepared thereby.

### Method for preparing deodorant composition

The method for preparing the deodorant composition of the present disclosure includes the step for irradiating a radiation onto a complex for removing a malodor-causing substance in the air.

### Complex

The complex includes a hydroxide of an alkali metal, an oxide of a Group 2 element, an oxide of a Group 4 element, and an oxide of a Group 13 element.

The type of the hydroxide of the alkali is not particularly limited thereto, but the type of the hydroxide of the alkali may include at least one hydroxide selected from the group consisting of a lithium (Li) hydroxide, a sodium (Na) hydroxide, and a potassium (K) hydroxide

The type of the oxide of the Group 2 element is not particularly limited thereto, but the type of the oxide of the Group 2 element may include at least one selected from the group consisting of a magnesium (Mg) oxide, and a calcium (Ca) oxide

The type of the oxide of the Group 4 element is not particularly limited thereto, but the type of the oxide of the Group 4 element may include at least one selected from the group consisting of a titanium (Ti) oxide, and a zirconium (Zr) oxide.

The type of the oxide of the Group 13 element is not particularly limited thereto, but the type of the oxide of the Group 13 element may include at least one selected from the group consisting of a boron (B) oxide, an aluminum (Al) oxide, and a gallium (Ga) oxide.

According to an embodiment, the hydroxide of the alkali metal may be NaOH, the oxide of the Group 2 element may be MgO, the oxide of the Group 4 element may be TiO₂, and the oxide of the Group 13 element may be Al₂O₃. However, this is provided only for the illustrative purpose. The type of a material included in the complex according to the present disclosure is not limited thereto. The complex according to the present disclosure should be interpreted as including various types of materials within a range exhibiting the excellent characteristic of removing the malodor-causing substance to be described above.

According to an embodiment, the complex may include, based on 100 parts by weight of the oxide of the Group 2 element, 50 to 300 parts by weight of the hydroxide of the alkali metal, 10 to 100 parts by weight of the oxide of the Group 4 element, and 10 to 200 parts by weight of the oxide of the Group 13 element. However, according to the present disclosure, the content of the material included in the complex is not limited to the above-described numerical range. According to the present disclosure, the content of each of the materials included in the complex may be interpreted as being varied within the range exhibiting the excellent characteristic of removing the malodor-causing substance to be described above.

According to an embodiment, the complex may further include oxides other than the hydroxide of the alkali metal, the oxide of the Group 2 element, the oxide of the Group 4 element, and the oxide of the Group 13 element to improve the characteristic of removing the malodor-causing substance. For example, the complex may further include at least one selected from the group consisting of a silicon (Si) oxide and an iron (Fe) oxide.

Each of the hydroxide of the alkali metal, the oxide of the Group 2 element, the oxide of the Group 4 element, and the oxide of the Group 13 element in the complex may have the excellent characteristic of removing the malodor-causing substance.

Specifically, each of the hydroxide of the alkali metal, the oxide of the Group 2 element, the oxide of the Group 13 element, and the oxide of the Group 4 element in the complex may absorb the moisture and the hydroxyl group-containing compound which are in contact with the composition.

In addition, the oxide of the Group 13 element in the complex may strongly absorb the moisture and the hydroxyl group-containing compound into the complex, thereby preventing the moisture and the hydroxyl group-containing compound from being re-discharged in the air. The oxide of the Group 4 element may adsorb a malodor-causing compound, such as a carbonyl group-containing compound (e.g., formaldehyde), an aromatic ring-containing compound (e.g., toluene, xylene, or benzene), and an ammonia-based compound, in addition to the moisture and the hydroxyl group-containing compound.

In addition, the hydroxide of the alkali metal in the complex may provide alkali metal ions and a hydroxyl group for oxidizing or reducing various malodor-causing compounds adsorbed to the complex, together with oxygen in the air continuously introduced into the complex. Accordingly, the hydroxide of the alkali metal may effectively decompose various malodor-causing compound adsorbed to the complex, and may prevent the various malodor-causing compound adsorbed to the complex from being re-emitted into the air.

Meanwhile, the complex may have the characteristic of removing the sulfur-based compound (e.g., hydrogen sulfide (H₂S)) among malodor-causing compounds. For example, the oxide (e.g., the oxide of the Group 13 element) and the hydroxyl groups (e.g., the hydroxyl groups in the alkali metal hydroxide) in complex is presumed to be oxidize hydrogen sulfide into a radical (H-S•) thereof together with oxygen in the air introduced into the complex. The radicals derived from the hydrogen sulfide react with each other to form hydrogen disulfide (H₂S₂), which reduces hydrogen sulfide.

### Irradiation of radiation

The inventors of the present disclosure have discovered that, when the radiation is irradiated onto the complex to form the deodorant composition, a removal rate of the formed deodorant composition for the malodor-causing compound (especially, a malodor-causing compound including a sulfur-based compound) is remarkably improved, as compared to a removal rate of the complex for the malodor-causing compound, through repeated studies. It is presumed that the result is made because, when the radiation is irradiated onto the complex, radicals having an excellent reactivity with the above-described sulfur-based compound may be formed in the complex (e.g., a hydroxy radical may be formed from the hydroxyl groups included in the alkali metal hydroxide in the complex), and the complex may serve as a radical trap for stably maintaining such radicals.

According to an embodiment, the radiation may be at least one selected from the group consisting of an electron beam, a gamma ray, an ultraviolet ray, an alpha ray, a beta ray, a neutron ray, and an X-ray. Preferably, the radiation may be an electron beam or a gamma ray, and more preferably, the radiation may be a gamma ray. In this case, when the radiation satisfies the above-condition, the radiation is irradiated into the complex to obtain the deodorant composition exhibiting the excellent characteristic of removing the malodor-causing compound (especially, a malodor-causing compound including a sulfur-based compound).

According to an embodiment, an irradiation dose of the radiation may range from 0.1 to 15 kGy, from 0.2 to 10 kGy, from 0.5 to 8.5 kGy, from 1.5 to 7.0 kGy, from 2.3 to 5.5 kGy, from 2.5 to 5.0 kGy, or from 2.8 to 4.3 kGy. In this case, when the irradiation dose of the radiation satisfies the above-described numerical ranges, the deodorant composition may have an excellent removal rate for the malodor-causing compound (especially, the malodor-causing compound including a sulfur-based compound), while securing sufficient economic efficiency in terms of costs based on the irradiation of the radiation.

As described above, when the deodorant composition is formed by irradiating the radiation onto the complex, the formed deodorant composition may have the excellent removal rate for the malodor-causing compound. In particular, the deodorant composition may have a significantly excellent removal rate for the malodor-causing compound including the sulfur-based compound. For example, the deodorant composition may have a significantly excellent removal rate for the sulfur-based compound designated as specified malodor substances under the Odor Prevention Act (e.g., including at least one selected from the group consisting of hydrogen sulfide, methyl mercaptan, dimethyl sulfide, and dimethyl disulfide). Accordingly, the deodorant composition may be used as a desulfurizing deodorant composition for effectively removing the sulfur-based compound.

### Deodorant composition

The deodorant composition according to the present disclosure was prepared through the method for preparing the deodorant composition, and may be formed by irradiating the radiation onto the complex. In the following description, the duplication of the complex and the radiation described in the method for preparing the deodorant composition will be omitted.

The deodorant composition may include the complex and may include a radical (e.g., a hydroxyl radical) formed by the irradiation of the radiation. Accordingly, the deodorant composition may have an excellent removal rate for odor-causing compound (especially, the deodorant composition including the sulfur-based compound) derived from the above radical.

The radical may include various types of radicals formed through the radiolysis of various substances in the complex as a result of the irradiation of the radiation. For example, the radical may include various types of radicals formed through the radiolysis of at least one selected from the group consisting of the hydroxide of the alkali metal, the oxide of the Group 2 element, the oxide of the Group 4 element, and the oxide of the Group 13 element, in the complex. As another example, the radical may include various types of radicals formed through the radiolysis of water or gas trapped in the complex. In this case, the complex may serve as a radical trap for stably maintaining various types of radicals. Accordingly, the radical may be stably maintained in the deodorant composition.

According to an embodiment, the radical may include hydroxyl radicals formed by the irradiation of the radiation onto the hydroxyl group included in the hydroxide of the alkali metal in the complex.

Meanwhile, as described above, the deodorant composition may be used for a desulfurizing deodorant composition for effectively removing the sulfur compound (especially, a sulfur compound designated as a specified malodor substance under the Odor Prevention Act).

Hereinafter, the present disclosure will be described in detail through an embodiment. However, these embodiments are provided only for the illustrative purpose, and the scope of the present disclosure is not limited to the embodiments even in any meaning.

### Example 1-1 to 1-9: deodorant compositions prepared by irradiating electron beam onto complex

1g of a complex including the hydroxide of the alkali metal, the oxide of the Group 2 element, the oxide of the Group 4 element, and the oxide of the Group 13 element (CAION proposed by WNC Co., Ltd.) was placed in a 15 mL conical tube, and then irradiated with electron beams (irradiation dose; see following Table 1).

In this case, the electron beam was irradiation by using an electron beam accelerator (Model ELV-8; 2.5 MeV; Eb-Tech, Korea) at the Advanced Radiation Research Institute, under the conditions of 2.5 MeV energy intensity and an acceleration current of 5 mA. The absorbed dose was measured using a B3 film dosimeter.

**[Table 1]**

| Example# | The irradiation (unit; kGy) of electron beam |
|---|---|
| Example 1-1 | 0.5 |
| Example 1-2 | 1.0 |
| Example 1-3 | 1.5 |
| Example 1-4 | 2.0 |
| Example 1-5 | 2.5 |
| Example 1-6 | 3.0 |
| Example 1-7 | 3.5 |
| Example 1-8 | 4.0 |
| Example 1-9 | 10 |

### Examples 2-1 to 2-9: Deodorant composition prepared by irradiating gamma ray onto complex

1g of a complex including the hydroxide of the alkali metal, the oxide of the Group 2 element, the oxide of the Group 4 element, and the oxide of the Group 13 element (CAION proposed by WNC Co., Ltd.) was placed in a 15 mL conical tube, and then irradiated with a gamma ray (irradiation dose; see following Table 2).

The irradiation of the gamma ray was performed using a cobalt-60 gamma ray irradiation facility having a source strength of 170,000 Ci at the Advanced Radiation Research Institute of the Korea Atomic Energy Research Institute (Jeongeup) (point source AECL, IR-79, MDS Nordion International Co. Ltd., Ottawa, ON, Canada), and an absorbed dose was verified using an alanine dosimeter (5 mm, Bruker Instruments, Rheinstetten, Germany) standardized in accordance with specifications of the International Atomic Energy Agency (IAEA).

**Table 2**

| Example# | The irradiation (unit; kGy) of gamma ray |
|---|---|
| Example 2-1 | 0.5 |
| Example 2-2 | 1.0 |
| Example 2-3 | 1.5 |
| Example 2-4 | 2.0 |
| Example 2-5 | 2.5 |
| Example 2-6 | 3.0 |
| Example 2-7 | 3.5 |
| Example 2-8 | 4.0 |
| Example 2-9 | 10 |

### Comparative Example 1: Complex not irradiated with radiation

The complex including the hydroxide of alkali metal, the oxide of the Group 2 element, the oxides of the Group 4 element, and the oxide of the Group 13 element (CAION proposed by WNC Co., Ltd.) was not irradiated with any radiation.

### Experimental Example 1: Determination of removal rate of sulfur-based compound over time

The removal rates of four sulfur compounds (hydrogen sulfide, methyl mercaptan, dimethyl sulfide, dimethyl disulfide) among regulated malodor substances was determined by using the deodorant compositions of Example 1-6, and Example 2-6, and the complex of Comparative Example 1.

### Experimental Example 1-1: Determination of removal rate of hydrogen sulfide over time

The deodorant composition of Example 1-6, the deodorant composition of Example 2-6, and the composite of Comparative Example 1, and hydrogen sulfide gas were injected into 3L Tedlar bag (polyvinyl fluoride provided by Top Trading ENG Co., Ltd.) and stirred by Stirrer (IS-971R, provided by J-Tech Co., Ltd.).

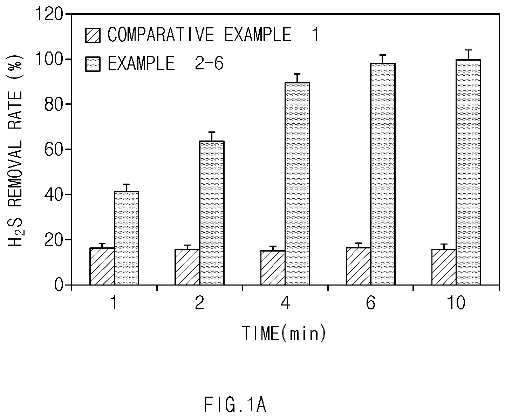

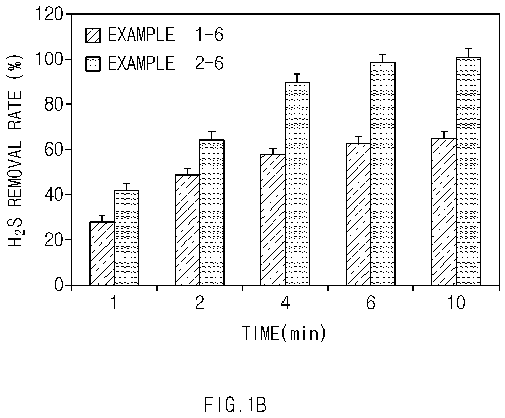

300 mL of gas was extracted from the Tedlar bag using a gas syringe when zero minute (that is, immediately after stirring), two minutes, four minutes, six minutes, or ten minutes were elapsed after stirring and introduced into a clean 500 mL Tedlar bag to prepare a sample. Then, the concentration of residual gas of the sample was measured and the measurement results are illustrated in FIGS. 1A and 1B.

In this case, the concentration of the residual gas of the hydrogen sulfide gas was analyzed using GC-MS (GC-MS-QP 2010 Ultra by Shimadzu), HP-1 (5 µm × 60 mm × 0.32 mm; Agilent Technologies Inc.) was used for a column. The temperature of an oven was stabilized to 80°C for 5 minutes, was increased at the rate of 10 °C/min for 12 minutes, and then was analyzed at 200°C for 7 minutes. Helium was used for carrier gas, and the flow rate was set to 1.5 mL/min.

Referring to FIGS. 1A and 1B, the deodorant compositions of Examples 1-6 and Examples 2-6 effectively removed hydrogen sulfide, as compared to the complex of Comparative Example 1. In particular, the deodorant composition of Example 2-6 showed a hydrogen sulfide removal rate of 99.5% within 10 minutes.

### Experimental Example 1-2: Determination of removal rate of methyl mercaptan over time

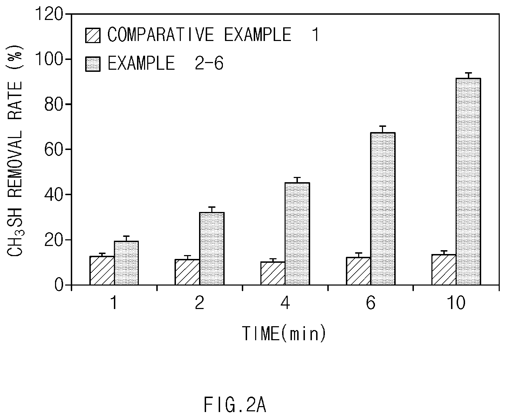

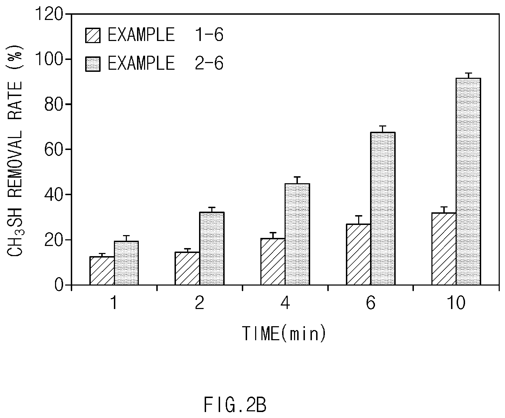

An experiment was performed in the same manner as that of Experimental Example 1-1, except that methyl mercaptan gas was used instead of hydrogen sulfide gas, when compared to Experimental Example 1-1, and the results are illustrated in FIGS. 2A and 2B.

Referring to FIGS. 2A and 2B, the deodorant compositions of Examples 1-6 and Examples 2-6 effectively removed methyl mercaptan, as compared to the complex of Comparative Example 1. In particular, the deodorant composition of Example 2-6 showed a hydrogen sulfide removal rate of 89.4% within 10 minutes.

### Experimental Example 1-3: Determination of removal rate of dimethyl sulfide over time

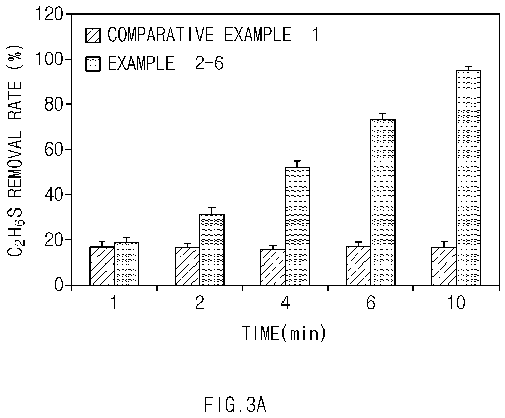

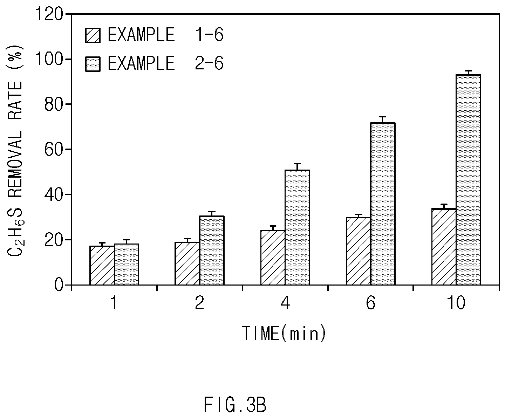

An experiment was performed in the same manner as that of Experimental Example 1-1, except that dimethyl sulfide was used instead of hydrogen sulfide gas, when compared to Experimental Example 1-1, and the results are illustrated in FIGS. 3A and 3B.

Referring to FIGS. 3A and 3B, the deodorant compositions of Examples 1-6 and Examples 2-6 effectively removed dimethyl sulfide, as compared to the complex of Comparative Example 1. In particular, the deodorant composition of Example 2-6 showed a hydrogen sulfide removal rate of 92.7% within 10 minutes.

### Experimental Example 1-4: Determination of removal rate of dimethyl disulfide over time

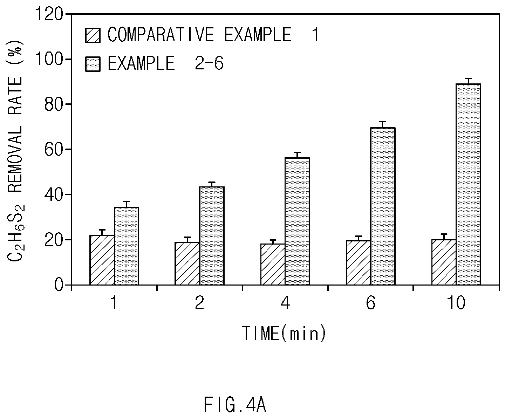

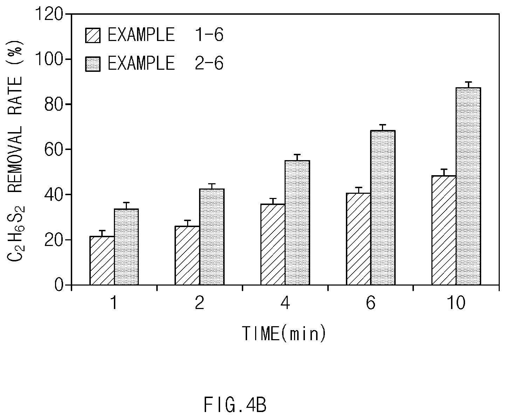

An experiment was performed in the same manner as that of Experimental Example 1-1, except that dimethyl disulfide gas was used instead of hydrogen sulfide gas, when compared to Experimental Example 1-1, and the results are illustrated in FIGS. 4A and 4B.

Referring to FIGS. 4A and 4B, the deodorant compositions of Examples 1-6 and Examples 2-6 effectively removed dimethyl disulfide, as compared to the complex of Comparative Example 1. In particular, the deodorant composition of Example 2-6 showed a dimethyl disulfide removal rate of 87.7% within 10 minutes.

### Experimental Example 2: Determination of removal rate of sulfur-based compound depending on irradiation dose of gamma ray

### Experimental Example 2-1: Determination of removal rate of hydrogen sulfide over time depending on irradiation dose of gamma ray

The deodorant composition of Example 2-1, the deodorant composition of Example 2-9, and hydrogen sulfide gas were injected into 3L Tedlar bag (polyvinyl fluoride provided by Top Trading ENG Co., Ltd.) and stirred by Stirrer (IS-971R, provided by Jeio-Tech Co., Ltd.).

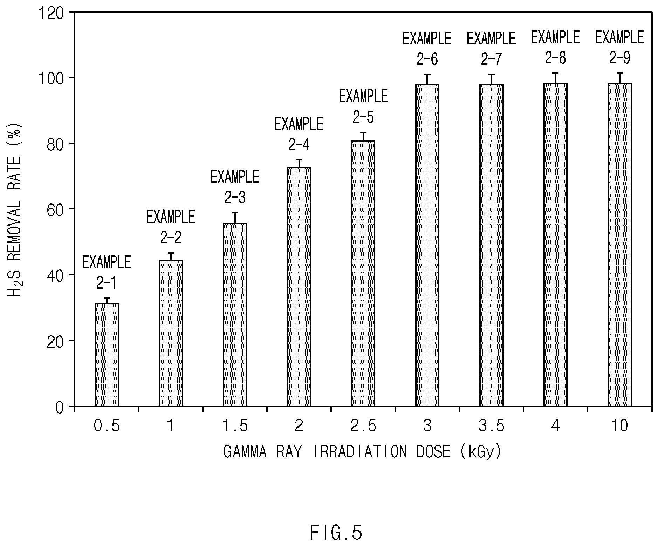

300 mL of gas was extracted from the Tedlar bag using a gas syringe when ten minutes were elapsed after stirring and introduced into a clean 500 mL Tedlar bag to prepare a sample. Then, the concentration of residual gas of the sample was measured and the measurement result is illustrated in FIG. 5.

In this case, the concentration of the residual gas of the hydrogen sulfide gas was analyzed using GC-MS (GC-MS-QP 2010 Ultra by Shimadzu), HP-1 (5 µm × 60 mm × 0.32 mm; Agilent Technologies Inc.) was used for a column. The temperature of an oven was stabilized to 80°C for 5 minutes, was increased at the rate of 10 °C/min for 12 minutes, and then was analyzed at 200°C for 7 minutes. Helium was used for carrier gas, and the flow rate was set to 1.5 mL/min.

Referring to FIG. 5, it may be confirmed that the increase of the irradiation dose of the gamma ray substantially increased the removal rate of the deodorant composition for the hydrogen sulfide (Examples 2-1 to 2-6) and then maintained the removal rate of the deodorant composition for the hydrogen sulfide to a specific level (Examples 2-6 to 2-9), and may be conformed that the most excellent removal rate for the hydrogen sulfide was exhibited when the gamma ray was irradiated in the irradiation dose of 3 kGy (Example 2-6).

### Experimental Example 2-2: Determination of removal rate of methyl mercaptan over time depending on irradiation dose of gamma ray

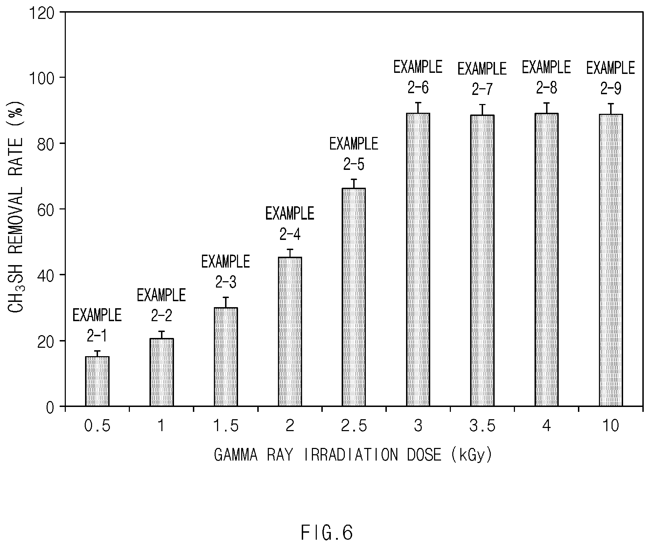

An experiment was performed in the same manner as that of Experimental Example 2-1, except that methyl mercaptan gas was used instead of hydrogen sulfide gas, when compared to Experimental Example 2-1, and the results are illustrated in FIGS. 6.

Referring to FIG. 6, it may be confirmed that the increase of the irradiation dose of the gamma ray substantially increased the removal rate of the deodorant composition for the methyl mercaptan (Examples 2-1 to 2-6) and then maintained the removal rate of the deodorant composition for the methyl mercaptan to a specific level (Examples 2-6 to 2-9), and may be conformed that the most excellent removal rate for the methyl mercaptan was exhibited when the gamma ray was irradiated in the irradiation dose of 3 kGy (Example 2-6).

### Experimental Example 2-3: Determination of removal rate of dimethyl sulfide over time depending on irradiation dose of gamma ray

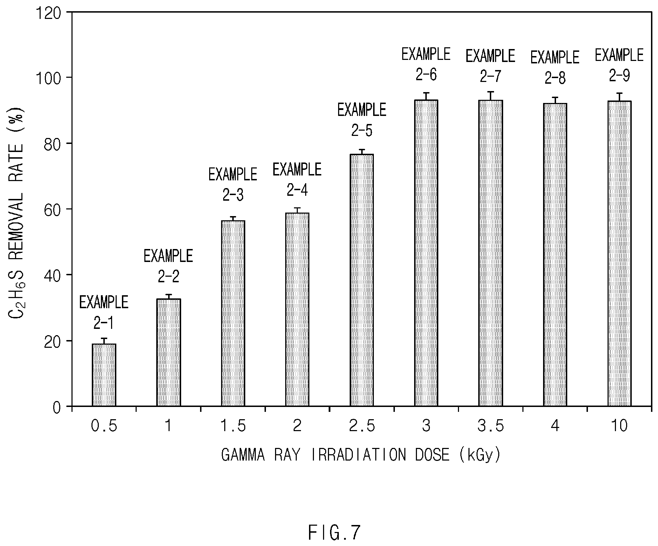

An experiment was performed in the same manner as that of Experimental Example 2-1, except that dimethyl sulfide gas was used instead of hydrogen sulfide gas, when compared to Experimental Example 2-1, and the results is illustrated in FIG. 7.

Referring to FIG. 7, it may be confirmed that the increase of the irradiation dose of the gamma ray substantially increased the removal rate of the deodorant composition for the dimethyl sulfide (Examples 2-1 to 2-6) and then maintained the removal rate of the deodorant composition for the dimethyl sulfide to a specific level (Examples 2-6 to 2-9), and may be conformed that the most excellent removal rate for the dimethyl sulfide was exhibited when the gamma ray was irradiated in the irradiation dose of 3 kGy (Example 2-6).

### Experimental Example 2-4: Determination of removal rate of dimethyl disulfide over time depending on irradiation dose of gamma ray

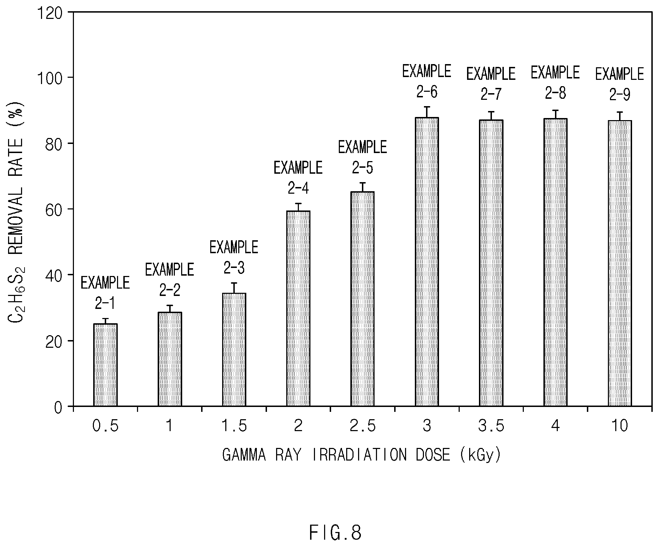

An experiment was performed in the same manner as that of Experimental Example 2-1, except that dimethyl disulfide gas was used instead of hydrogen sulfide gas, when compared to Experimental Example 2-1, and the results is illustrated in FIG. 8.

Referring to FIG. 8, it may be confirmed that the increase of the irradiation dose of the gamma ray substantially increased the removal rate of the deodorant composition for the dimethyl disulfide (Examples 2-1 to 2-6) and then maintained the removal rate of the deodorant composition for the dimethyl disulfide to a specific level (Examples 2-6 to 2-9), and may be conformed that the most excellent removal rate for the dimethyl disulfide was exhibited when the gamma ray was irradiated in the irradiation dose of 3 kGy (Example 2-6).

### Experimental Example 3: Measurement of radical concentration in deodorant composition based on irradiation dose of radiation

After irradiating a radiation onto the deodorant composition of examples and standing the deodorant composition for two weeks, a radical measurement manner using pyrogallol as a substrate was performed to measure a concentration of radicals in the deodorant composition.

In this case, pyrogallol, which has a maximum UV absorbance at 280 nm, reacts with radicals such that pyrogallol is switched into a material having a maximum UV absorbance at 360 nm. In this case, an optical density (o.d.) of the switched material may be measured to quantify an amount of the radicals.

### Experimental Example 3-1: Measurement of radical concentration in deodorant composition based on irradiation dose of electron beam

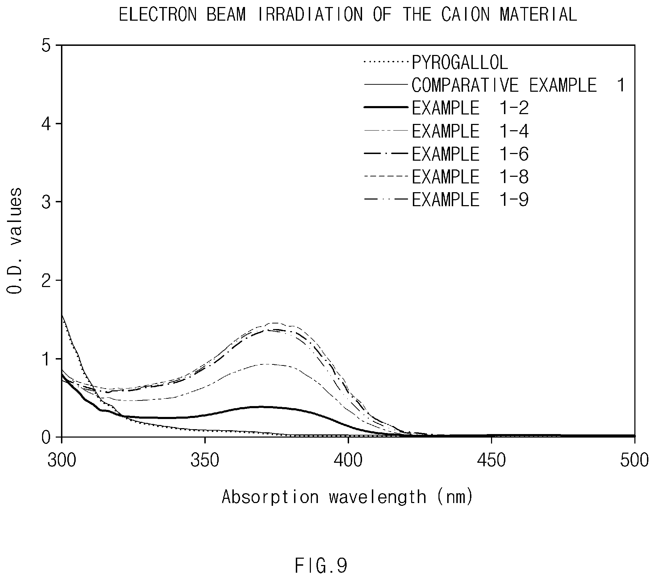

1 mL of the deodorant compositions of Examples 1-2, 1-4, 1-6, 1-8, and 1-9 and 1 mL of the complex of Comparative Example 1 were induced a radical reaction with 1 mg of pyrogallol at room temperature for 5 hours. Then, the result was introduced into a quartz cell having a volume of 1 mL. Then, the optical density was measured in the wavelength range from 300 nm to 500 nm using ELISA reader (Infinite F200; Tecan Austria GmBH; Grodig; Austria). The result is shown in FIG. 9.

Referring to FIG. 9, it may be confirmed that a content of the radical reaction product was increased, as an irradiation dose of the electron beam was increased (Comparative Example 1, Examples 1-2, 1-4, and 1-6), and may be confirmed that the content of the radical reaction product was substantially uniform, when the irradiation dose of the electron beam was at least a specific level (Examples 1-6, 1-8, and 1-9). In other words, it may be confirmed that the content of radicals in the deodorant composition generally was increased, as the irradiation dose of the electron beam irradiated onto the complex was increased.

### Experimental Example 3-2: Measurement of radical concentration in deodorant composition based on irradiation dose of gamma ray

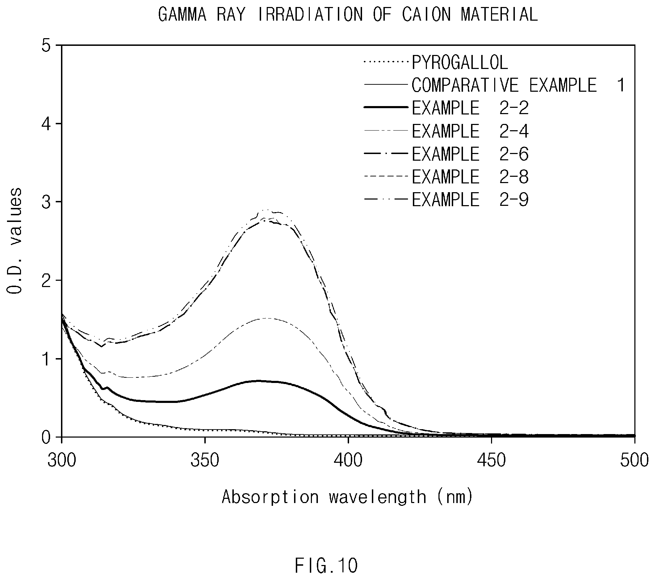

The experiment was performed in the same manner as that of Experimental Example 3-1 except that 1 mL of deodorant composition of Examples 2-2, 2-4, 2-6, 2-8, or 2-9 was used instead of 1 mL of the deodorant composition of Examples 1-2, 1-4, 1-6, 1-8, or 1-9, when compared Experimental Example 3-1, and the result is illustrated in FIG. 10.

Referring to FIG. 10, it may be confirmed that a content of the radical reaction product was increased, as an irradiation dose of the gamma ray was increased (Comparative Example 1, Examples 2-2, 2-4, and 2-6), and may be confirmed that the content of the radical reaction product was substantially uniform, when the irradiation dose of the gamma ray was at least a specific level (Examples 2-6, 2-8, and 2-9). In other words, it may be confirmed that the content of radicals in the deodorant composition generally was increased, as the irradiation dose of the gamma ray irradiated onto the complex was increased.

### Experimental Example 4: Measurement of radical concentration in deodorant composition depending on standing time after irradiating radiation

### Experimental Example 4-1: Measurement of radical concentration in deodorant composition depending on standing time after irradiating electron beam

After storing the deodorant composition of Example 1-6 for 0 days (that is, immediately after preparing the deodorant composition of Example 1-6), 10 days, 20 days, and 30 days, the radical concentration was measured using an ESR spectrometer. In addition, after storing the deodorant composition of, Comparative Example 1 for 0 days (that is, immediately after preparing the deodorant composition of Comparative Example 1), 10 days, 20 days, and 30 days, the radical concentration was measured using the ESR equipment.

Specifically, an ESR spectrum was measured with respect to a sample of the deodorant composition of Example 1-6 or the complex of Comparative Example 1 at room temperature under conditions of a power of 1 mW, a modulation frequency of 9.65 GHz, and a magnetic field of 335 ± 8 mT using a quartz ESR tube (5 mm × 200 mm) and an ESR spectrometer (JES-X310 ESR spectrometer; JEOL; Japan), and a radical concentration derived from the result is shown in following Table 3.

**[Table 3]**

| Standing time | Radical concentration (Example 1-6) | Radical concentration (Comparative Example 1) |
|---|---|---|
| Zero days | 6.9(± 1.5)% | 0.01(±0.001)% |
| 10 days | 5.5(±1.2)% | 0.01(± 0.001)% |
| 20 days | 5.9(±1.1)% | 0.01(± 0.001)% |
| 30 days | 6.1(±1.4)% | 0.01(±0.001)% |

Referring to FIG. 3, the deodorant composition of Example 1-6 included radicals at a specific concentration, when compared to the complex of Comparative Example 1 not irradiated with the radiation. In this case, it may be confirmed that the concentration of radicals in the deodorant composition of Example 1-6 was generally constant, regardless of a standing time of the deodorant composition of Example 1-6. In other words, it may be confirmed that radicals in the deodorant composition of Example 1-6, which was formed through the irradiation of the electron beam, were stably maintained (the deodorant composition effectively served as a radical trap).

### Experimental Example 4-2: Measurement of radical concentration in deodorant composition depending on standing time after irradiating gamma ray

The experiment was performed in the same manner as that of Experimental Example 4-1 except that the deodorant composition of Example 2-6 was used instead of the deodorant composition of Example 1-6, when compared to Experimental Example 4-1.

**[Table 4]**

| Standing time | Radical concentration (Example 2-6) | Radical concentration (Comparative Example 1) |
|---|---|---|
| 0 days | 13.2(±2.3)% | 0.01(±0.001)% |
| 10 days | 10.3(±3.5)% | 0.01(±0.001)% |
| 20 days | 12.3(±1.9)% | 0.01(±0.001)% |
| 30 days | 16.4(±3.7)% | 0.01(±0.001)% |

Referring to FIG. 4, the deodorant composition of Example 2-6 included radicals at a specific concentration, when compared to the complex of Comparative Example 1 not irradiated with the radiation. In this case, it may be confirmed that the concentration of radicals in the deodorant composition of Example 2-6 was generally constant, regardless of a standing time of the deodorant composition of Example 2-6. In other words, it may be confirmed that radicals in the deodorant composition of Example 2-6, which was formed through the irradiation of the gamma ray, were stably maintained (the deodorant composition effectively served as a radical trap).

## Claims

1. A method for preparing a deodorant composition, the method comprising:
irradiating a radiation onto a complex including a hydroxide of an alkali metal, an oxide of a Group 2 element, an oxide of a Group 4 element, and an oxide of a Group 13 element.

2. The method of claim 1, wherein the deodorant composition is a desulfurizing deodorant composition for removing a sulfur-based compound.

3. The method of claim 2, wherein the sulfur-based compound includes at least one selected from the group consisting of hydrogen sulfide, methyl mercaptan, dimethyl sulfide, and dimethyl disulfide.

4. The method of claim 1, wherein the radiation is at least one selected from the group consisting of an electron beam, a gamma ray, an ultraviolet ray, an alpha ray, a beta ray, a neutron ray, and an X-ray.

5. The method of claim 1, wherein an irradiation dose of the radiation ranges from 0.1 kGy to 15 kGy.

6. The method of claim 1, wherein the hydroxide of the alkali metal includes at least one hydroxide selected from a lithium (Li) hydroxide, a sodium (Na) hydroxide, and a potassium (K) hydroxide,
wherein the oxide of the Group 2 element includes at least one oxide selected from a magnesium (Mg) oxide and a calcium (Ca) oxide,
wherein the oxide of the Group 4 element includes at least one oxide selected from a titanium (Ti) oxide and a zirconium (Zr) oxide, and
wherein the oxide of the Group 13 element includes at least one oxide selected from a boron (B) oxide, an aluminum (Al) oxide, and a gallium (Ga) oxide.

7. The method of claim 1, wherein the complex further includes at least one oxide selected from the group consisting of a silicon (Si) oxide and an iron (Fe) oxide.

8. The method of claim 1, wherein the hydroxide of the alkali metal is NaOH,
wherein the oxide of the Group 2 element is MgO,
wherein the oxide of the Group 4 element is TiO₂, and
wherein the oxide of the Group 13 element is Al₂O₃.

9. The method of claim 1, wherein the complex includes, based on 100 parts by weight of the oxide of the Group 2 element, 50 to 300 parts by weight of the hydroxide of the alkali metal, 10 to 100 parts by weight of the oxide of the Group 4 element, and 10 to 200 parts by weight of the oxide of the Group 13 element.

10. A deodorant composition comprising:
a complex including a hydroxide of an alkali metal, an oxide of a Group 2 element, an oxide of a Group 4 element, and an oxide of a Group 13 element, and
wherein the composition includes radicals.

11. The deodorant composition of claim 10, wherein the deodorant composition is a desulfurizing deodorant composition for removing a sulfur-based compound.

12. The deodorant composition of claim 11, wherein the sulfur-based compound includes: at least one selected from the group consisting of hydrogen sulfide, methyl mercaptan, dimethyl sulfide, and dimethyl disulfide.

13. The deodorant composition of claim 10, wherein the radicals are included in at least one selected from the group consisting of the hydroxide of the alkali metal, the oxide of the Group 2 element, the oxide of the Group 4 element, and the oxide of the Group 13 element.

14. The deodorant composition of claim 13, wherein the radicals include hydroxy radicals included in the hydroxide of the alkali metal.

15. The deodorant composition of claim 10, wherein the hydroxide of the alkali metal includes at least one hydroxide selected from a lithium (Li) hydroxide, a sodium (Na) hydroxide, and a potassium (K) hydroxide,
wherein the oxide of the Group 2 element includes at least one oxide selected from a magnesium (Mg) oxide and a calcium (Ca) oxide,
wherein the oxide of the Group 4 element includes at least one oxide selected from a titanium (Ti) oxide and a zirconium (Zr) oxide, and
wherein the oxide of the Group 13 element includes at least one oxide selected from a boron (B) oxide, an aluminum (Al) oxide, and a gallium (Ga) oxide.

16. The deodorant composition of claim 10, wherein the complex further includes at least one oxide selected from the group consisting of a silicon (Si) oxide and an iron (Fe) oxide.

17. The deodorant composition of claim 10, wherein the hydroxide of the alkali metal is NaOH,
wherein the oxide of the Group 2 element is MgO,
wherein the oxide of the Group 13 element is Al₂O₃, and
wherein the oxide of the Group 4 element is TiO₂.

18. The deodorant composition of claim 10, wherein the complex includes, based on 100 parts by weight of the oxide of the Group 2 element, 50 to 300 parts by weight of the hydroxide of the alkali metal, 10 to 100 parts by weight of the oxide of the Group 4 element, and 10 to 200 parts by weight of the oxide of the Group 13 element.

19. The deodorant composition of claim 10, wherein the deodorant composition has a powder form, a film form, or a block form.
